(19) Europäisches Patentamt
European Patent Office
Office européen des brevets

(11) Publication number: **0 206 635**
**B1**

(12) **EUROPEAN PATENT SPECIFICATION**

(45) Date of publication of the patent specification: **15.11.89**

(51) Int. Cl.⁴: **C 07 C 101/72**, C 07 C 79/46, C 07 C 99/00, C 07 C 76/02

(21) Application number: **86304435.0**

(22) Date of filling: **10.06.86**

(54) **Preparation of 3-amino-4-hydroxybenzoic acids.**

(30) Priority: **26.06.85 US 749079**
**10.02.86 US 827996**

(43) Date of publication of application:
**30.12.86 Bulletin 86/52**

(45) Publication of the grant of the patent:
**15.11.89 Bulletin 89/46**

(84) Designated Contracting States:
**AT BE CH DE FR GB IT LI LU NL SE**

(56) References cited:
**FR-A-2 097 808**

**BEILSTEINS HANDBUCH DER ORGANISCHEN CHEMIE, ed. 4, vol. XIV, 1931, "Isocyclische Reihe", VERLAG JULIUS SPRINGER, Berlin, p. 593**
**CESARE FERRI "Reaktionen der organischen Synthese", 1978, GEORG THIEME VERLAG, Stuttgart, p. 200**
**METHODEN DER ORGANISCHEN CHEMIE (HOUBEN-WEYL), ed. 4, vol. XI/1, 1957, "Stickstoffverbindungen II, Amine, 1. Herstellung", GEORG THIEME VERLAG, Stuttgart, pp. 374-382**

(73) Proprietor: **THE DOW CHEMICAL COMPANY, 2030 Dow Center Abbott Road P.O. Box 1967, Midland Michigan 48640- 1967 (US)**

(72) Inventor: **Lysenko, Zenon, 214 West Meadowbrook Drive, Midland Michigan 48640 (US)**

(74) Representative: **Burford, Anthony Frederick, W.H. Beck, Greener & Co. 7 Stone Buildings Lincoln's Inn, London WC2A 3SZ (GB)**

**EP 0 206 635 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1989

## Description

This invention concerns a process for the preparation of 3-amino-4-hydroxybenzoic acids.

Papenfuss, U.S. Patent 3 929 864, discloses a process for the preparation of 4-hydroxy-3-nitrobenzoic acid alkyl esters wherein 4-hydroxybenzoic alkyl esters are contacted with nitric acid at about 0°C to 60°C wherein the nitric acid has a strength of 30 to 62 percent by weight so as to prepare 4-hydroxy-3-nitrobenzoic acid alkyl esters. These compounds can be used directly for further reactions, for example, subjected to catalytic hydrogenation to give 4-hydroxy-3-aminobenzoic acid esters. The Papenfuss process suffers from the drawback of over-nitration and of decarboxylation of the starting material. Purification of the product of the Papenfuss process is difficult and tedious.

Beilstein's Handbuch Ed. 4, vol XIV (1931) page 593 discloses that 3-amino-4-hydroxybenzoic acid can be prepared by the reduction of 3-nitro-4-hydroxybenzoic acid using tin and hydrochloric acid.

FR 2097808 discloses the preparation of 3-nitro-4-chlorobenzoic acid sodium salt by sodium hydroxide hydrolysis of a corresponding lower alkyl ester obtained by nitric acid/sulfuric acid nitration of the corresponding 4-chlorobenzoic acid lower alkyl ester.

3-Amino-4-hydroxybenzoic acids are useful in the preparation of AB polybenzoxazole ordered polymers. Previous methods used to prepare such compounds afford such compounds in low yields requiring extensive purification so that they may be rendered suitable for polymerization. It has been discovered that the presence of impurities in 3-amino-4-hydroxybenzoic acids prevent the formation of high molecular weight AB polybenzoxazoles. This invention provides a process which prepares 3-amino-4-hydroxybenzoic acids in high yields with high purity.

This invention is directed to a process for the preparation of a 3-amino-4-hydroxybenzoic acid which comprises:

(a) contacting a p-halobenzoic acid or ester thereof, which is unsubstituted or substituted with one or two alkyl groups, with the proviso that at least one of the positions ortho to the halo moiety is unsubstituted, with nitric acid in an acidic reaction medium under conditions such that 3-nitro-4-halobenzoic acid or ester thereof is prepared;

(b) contacting the 3-nitro-4-halobenzoic acid or ester thereof with at least a two equivalent excess of an alkali metal hydroxide in a reaction medium under conditions such that the halo moiety is replaced with a hydroxide moiety or a salt thereof, to prepare a 3-nitro-4-hydroxybenzoic acid, or salt thereof; and

(c) reducing the 3-nitro-4-hydroxy-benzoic acid or salt thereof in a reaction medium under conditions such that a 4-hydroxy-3-aminobenzoic acid is prepared.

The process of this invention results in the surprising preparation of 3-amino-4-hydroxy-benzoic acids in high yield and very high purity. Such highly pure products are useful in the preparation of high molecular weights polybenzoxazoles without extensive and difficult purification.

In the first step of the process, a p-halobenzoic acid or ester thereof which is unsubstituted or substituted with one or two alkyl groups is contacted with nitric acid in an acidic reaction medium to prepare a 3-nitro-4-halobenzoic acid or ester thereof. Suitable starting materials include any p-halobenzoic acid or ester thereof, wherein the benzene ring is unsubstituted or substituted with one or two alkyl groups, provided that at least one of the positions ortho to the halo moiety is unsubstituted. "Halo" refers herein to chlorine, bromine, iodine and fluorine. Preferred p-halobenzoic acids or esters thereof correspond to the formula

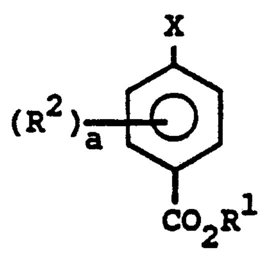

(I)

wherein

$R^1$ is hydrogen or alkyl;

$R^2$ is independently in each occurrence alkyl;

X is a halogen; and

a is 0, 1 or 2,

with the proviso that at least one of the positions ortho to the halo moiety is unsubstituted.

Examples of p-halobenzoic acids or esters thereof useful in this invention include p-chloro-2-methylbenzoic acid, p-chloro-2-ethylbenzoic acid, p-chloro-2-propylbenzoic acid, p-bromo-2-methylbenzoic acid, p-bromo-2-ethylbenzoic acid, p-bromo-2-propylbenzoic acid, methyl p-chloro-2-methylbenzoate, methyl p-chloro-2-ethylbenzoate, methyl p-chloro-2-propylbenzoate, ethyl p-chloro-2-methylbenzoate, ethyl p-chloro-2-ethylbenzoate, ethyl p-chloro-2-propylbenzoate, propyl p-chloro-2-methylbenzoate, propyl p-chloro-2-ethylbenzoate, propyl p-chloro-2-propylbenzoate, methyl p-bromo-3-methylbenzoate, methyl p-bromo-3 -ethylbenzoate, methyl p-bromo-3-propylbenzoate, ethyl p-bromo-3-methylbenzoate, ethyl p-bromo-3-ethylbenzoate, ethyl p-bromo-3-propylbenzoate, propyl p-bromo-3-methylbenzoate, propyl p-bromo-3-ethylbenzoate, and propyl p-bromo-3-propylbenzoate. Preferred are the p-halobenzoic acids which do not have alkyl substituents with p-chlorobenzoic acid being most preferred.

The products prepared by this step are the 3-nitro-4-halobenzoic acids or esters thereof which may be unsubstituted or further substituted with one or two alkyl groups. Preferred 3-nitro-4-halobenzoic acids or esters thereof correspond to the formula

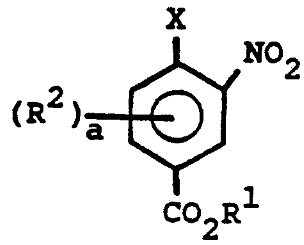

(II)

wherein $R^1$, $R^2$, a and X are as hereinbefore defined.

Examples of preferred 3-nitro-4-halobenzoic acids or esters thereof include 3-nitro-4-chlorobenzoic acid, 3-nitro-4-bromobenzoic acid, 3-nitro-4-chloro-2-metyhylbenzoic acid, 3-nitro-4-chloro-2-ethylbenzoic acid, 3-nitro-4-chloro-2-propylbenzoic acid, 3-nitro-4-chloro-5-methylbenzoic acid, 3-nitro-4-chloro-5-ethylbenzoic acid, 3-nitro-4-chloro-5-propylbenzoic acid, 3-nitro-4-bromo-2-methylbenzoic acid, 3-nitro-4-bromo-2-ethylbenzoic acid, 3-nitro-4-bromo-2-propybenzoic acid, 3-nitro-4-bromo-5-methylbenzoic acid, 3-nitro-4-bromo-5-ethylbenzoic acid, 3-nitro-4-bromo-5-propylbenzoic acid, methyl 3-nitro-4-chloro-2-methylbenzoate, methyl 3-nitro-4-chloro-2-ethylbenzoate, methyl 3-nitro-4-chloro-2-propylbenzoate, ethyl 3-nitro-4-chloro-2-methylbenzoate, ethyl 3-nitro-4-chloro-2-ethylbenzoate, ethyl 3-nitro-4-chloro-2-propylbenzoate, propyl 3-notro-4-chloro-2-methylbenzoate, propyl 3-nitro-4-chloro-2-ethylbenzoate, propyl 3-nitro-4-chloro-2-propylbenzoate, methyl 3-nitro-4-chloro-5-methylbenzoate, methyl 3-nitro-4-chloro-5-ethylbenzoate, methyl 3-nitro-4-chloro-5-propyl-3-nitro-4-chloro-5-propylbenzoate, ethyl 3-nitro-4-chloro-5-methyl-benzoate, ethyl 3-nitro-4-chloro-5-ethylbenzoate, ethyl 3-nitro-4-chloro-5-propylbenzoate, propyl 3-nitro-4-chloro-5-methylbenzoate, propyl 3-nitro-4-chloro-5-ethylbenzoate, propyl 3-nitro-4-chloro-5-propylbenzoate, methyl 3-nitro-4-bromo-2-methylbenzoate, methyl 3-nitro-4-bromo-2-ethylbenzoate, methyl 3-nitro-4-bromo-2-propylbenzoate, ethyl 3-nitro-4-bromo-2-methyl-benzoate, ethyl 3-nitro-4-bromo-2-ethylbenzoate, ethyl 3-nitro-4-bromo-2-propylbenzoate, propyl 3-nitro-4-bromo-2-methylbenzoate, propyl 3-nitro-4-bromo-2-ethylbenzoate, propyl 3-nitro-4-bromo-2-propylbenzoate, methyl 3-nitro-4-bromo-5-mrthylbenzoate, methyl 3-nitro-4-bromo-5-ethylbenzoate, methyl 3-nitro-4-bromo-5-propylbenzoate, ethyl 3-nitro-4-bromo-5-methylbenzoate, ethyl 3-nitro-4-bromo-5-ethylbenzoate, ethyl 3-nitro-4-bromo-5-propylbenzoate, propyl 3-nitro-4-bromo-5-methylbenzoate, propyl 3-nitro-4-bromo-5-ethylbenzoate, propyl 3-nitro-4-bromo-5-propylbenzoate, 3-nitro-4-chloro-6-methylbenzoic acid, 3-nitro-4-chloro-6-ethylbenzoic acid, 3-nitro-4-chloro 6-propylbenzoic acid, 3-nitro-4-bromo-6-methylbenzoic acid, 3-nitro-4-bromo-6-ethylbenzoic acid, 3-nitro-4-bromo-6-propylbenzoic acid, methyl 3-nitro-4-chloro-6-methylbenzoate, methyl 3-nitro-4-chloro-6-ethylbenzoate, methyl 3-nitro-4-chloro-6-propylbenzoate, ethyl 3-nitro-4-chloro-6-methylbenzoate, ethyl 3-nitro-4-chloro-6-ethylbenzoate, ethyl 3-nitro-4-chloro-6-propylbenzoate, propyl 3-nitro-4-chloro-6-methylbenzoate, propyl 3-nitro-4-chloro-6-ethylbenzoate, and propyl 3-nitro-4-chloro-6-propylbenzoate. Preferred are the 3-nitro-4-halobenzoic acids with 3-nitro-4-chlorobenzoic acid being most preferred.

The process of step 1 can be exemplified by the following equation

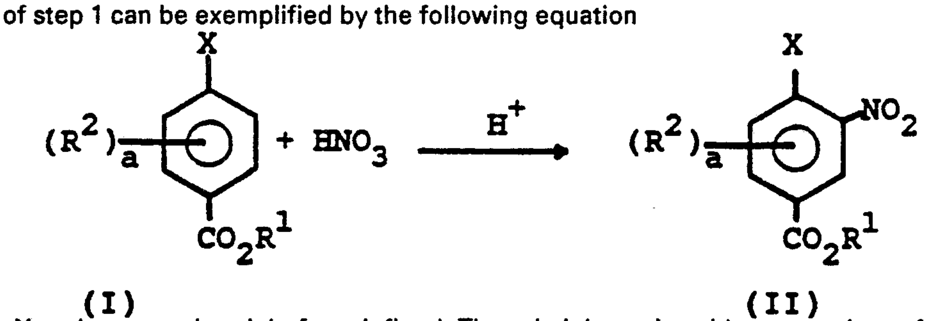

wherein $R^1$, $R^2$, X and a are as hereinbefore defined. The p-halobenzoic acid or ester thereof is reacted with a sufficient amount of nitric acid so as to prepare the desired 3-nitro-4-halobenzoic acid or ester thereof. Preferably, at least about one equivalent of nitric acid per equivalent of p-halobenzoic acid or ester thereof is used. A slight excess of nitric acid is desirable so as to drive the reaction to completion with respect to the p-halobenzoic acid or ester thereof. This process is performed in the presence of an acidic reaction medium.

An acidic reaction medium is a liquid medium which allows at least some nitration to occur upon the agitated contact of (1) 1 liter of acidic reaction medium, (2) 500 g of powdered (1 - 2 micrometers) p-chlorobenzoic acid and 1 drop of 1.0 N nitric acid at atmospheric pressure and a temperature of 30°C. Preferably, the acidic reaction medium contains more than about 20 weight percent of a Lewis or Bronsted acid, based on the weight of the acidic reaction medium; more preferably more than about 50 weight percent, even more preferably more than about 70 weight percent and most preferably more than about 90 weight percent. Preferably, the

Lewis or Bronsted acid is a Bronsted acid, more preferably $H_2SO_4$, HCl, HOAc, $H_3PO_4$, $CH_3SO_3H$, fuming nitric acid, HBr HF, HI and the like and most preferably $H_2SO_4$. Preferably, the $H_2SO_4$ is in excess. Excess sulfuric acid means greater than about 1 equivalent of sulfuric acid per equivalent of p-halobenzoic acid, preferably the ratio of sulfuric acid to p-halobenzoic acid or ester thereof is 1.3 : 1 or greater. Any remainder of the acid reaction medium, that is any nonacid portion of the acid reaction medium, is anything which allows the formation of an acid reaction medium. Preferably, the remainder is an organic liquid or water with water being most preferred.

In some instances the p-halobenzoic acid or ester thereof is insoluble in a given acidic reaction medium. If this is the case, it is preferred that the p-halobenzoic acid or ester thereof be ground into a powder (1 - 2 micrometers) and suspended in the reaction medium.

This process can be run at any temperature at which the desired product is prepared. Preferable temperatures are between -10°C and 50°C, with between -6°C and 40°C being more preferred, with between 15°C and 40°C being most preferred.

The reactants are contacted for a time sufficient to allow the desired formation of 3-nitro-4-halobenzoic acid or ester thereof. Preferable reaction times are between 2 and 48 hours, with between 9 and 36 hours being most preferred.

The 3-nitro-4-halobenzoic acid or ester thereof can be recovered by diluting the reaction medium with a small amount of water, preferably between 25 and 100 percent by volume, and more preferably 25 percent by volume, so as to result in complete precipitation of the desired product. The product can thereafter be recovered by filtration, and thereafter washed with water. The product may optionally be dried by any means well-known in the art, although drying is not necessary.

The 3-nitro-4-halobenzoic acid, or ester thereof, so prepared is thereafter contacted with an alkali metal hydroxide in a reaction medium so as to replace the halo moiety with a hydroxide moiety or a salt thereof, therefore preparing a 3-nitro-4-hydroxybenzoic acid, or salt thereof. Such 3-nitro-4-hydroxybenzoic acid or salt thereof, can further be substituted by one or two alkyl groups. Preferred 3-nitro-4-hydroxybenzoic acids, or salts thereof, correspond to the formula

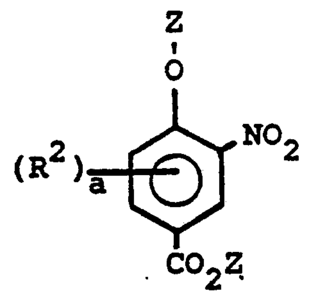

(III)

wherein $R^1$, $R^2$ and a are as hereinbefore defined, and Z is independently in each occurrence hydrogen or a cation.

Examples of preferred 3-nitro-4-hydroxybenzoic acids, or salts thereof include 3-nitro-4-hydroxybenzoic acid, 2-methyl-3-nitro-4-hydroxybenzoic acid, 5-methyl-3-nitro-4-hydroxybenzoic acid, 6-methyl-3-nitro-4-hydroxybenzoic acid, 2-ethyl-3-nitro-4-hydroxybenzoic acid, 5-ethyl-3-nitro-4-hydroxybenzoic acid, 6-ethyl-3-nitro-4-hydroxybenzoic acid, 2-propyl-3-nitro-4-hydroxybenzoic acid, 5-propyl-3-nitro-4-hydroxybenzoic acid and 6-propyl-3-nitro-4-hydroxybenzoic acid, or alkali earth metal salts thereof. The preferred acid is the 3-nitro-4-hydroxybenzoic acid.

The process of this step is generally represented by the equation

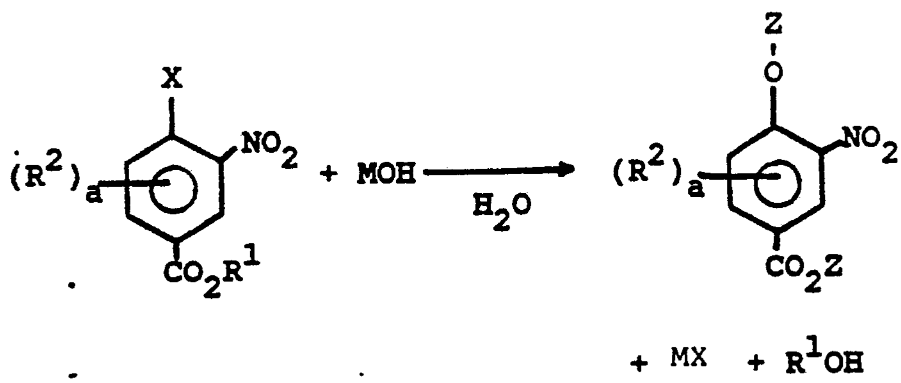


(II) (III)

wherein M is an alkali metal, and $R^1$, $R^2$, X, Z and a are as hereinbefore defined. The 3-nitro-4-halobenzoic acid or ester thereof is reacted with at least a 2 equivalent excess of an alkali metal hydroxide. In that embodiment

wherein $R^1$ is hydrogen, wherein the starting material is a benzoic acid, 3 equivalents or more of alkali metal hydroxide is suitable. In that embodiment where $R^1$ is an alkyl group, that is, wherein the starting material is a benzoate ester, at least 4 equivalents of alkali metal hydroxide is preferred. The process can he run with any amount of alkali metal hydroxide which gives the desired product, but it has been discovered that at least a 1 to 4 equivalent excess results in driving the reaction to completion with respect to the benzoic acid, or benzoate ester, respectively. Wherein the starting material is a benzoic acid, it is preferred to use between about 4.5 and 5.5 equivalents of alkali metal hydroxide for each equivalent of benzoic acid. Wherein the starting material is a benzoate ester, it is preferred to use between about 5.5 and 6.5 equivalents of alkali metal hydroxide for each equivalent of benzoate ester. Preferred alkali metal hydroxides are sodium hydroxide, potassium hydroxide, lithium hydroxide, cesium hydroxide and rubidium hydroxide with sodium hydroxide being most preferred.

This process takes place in a reaction medium. A sufficient amount of reaction medium which allows the reactants to react may be used. Preferable reaction media include inert solvents and water, with water being more preferred.

This process is performed at any temperature at which the desired product is formed. Preferred temperatures are between 65°C and 120°C, more preferably higher than 90°C and/or less than 110°C, and about 103°C being most preferred. In the most preferred embodiment, the reaction is run at reflux in water. The product may be recovered by contacting the reaction medium with a sufficient amount of strong protic mineral acid to result in a 3-12N solution, preferably a 6-12N solution of the acid. Suitable acids include hydrochloric acid, hydrobromic acid, sulfuric acid, nitric acid, and the like. The preferred acid is hydrochloric acid. The product is recovered in the acidic form as it precipitates from solution.

In the third step of the process of this invention, the 3-nitro-4-hydroxybenzoic acid, or salt thereof, is reduced to the 3-amino-4-hydroxybenzoic acid. The product is a 3-amino-4-hydroxybenzoic acid, which can be substituted with one or two alkyl groups. 3-Amino-4-hydroxybenzoic acid as referred to herein further refers to the amine salts of the 3-amino-4-hydroxybenzoic acid, as such compound is generally recovered in the amine salt form. Preferred 3-amino-4-hydroxyben zoic acids correspond to the formula

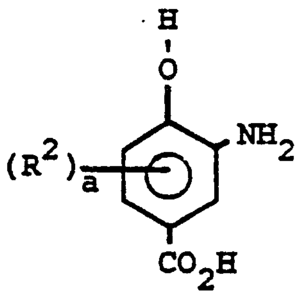

(IV)

wherein $R^2$ and a are as hereinbefore defined.

Examples of preferred 3-amino-4-hydroxybenzoic acids prepared by this process include 3-amino-4-hydroxybenzoic acid, 2-methyl-3-amino-4-hydroxybenzoic acid, 5-methyl-3-amino-4-hydroxybenzoic acid, 6-methyl-3-Amino-4-hydroxybenzoic acid, 2-ethyl-3-amino-4-hydroxybenzoic acid, 5-ethyl-3-amino-4-hydroxybenzoic acid, 6-ethyl-3-amino-4-hydroxybenzoic acid, 2-propyl-3-amino-4-hydroxybenzoic cid, 5-propyl-3-amino-4-hydroxybenzoic acid and 6-propyl-3-amino-4-hydroxybenzoic acid. The preferred species is a 3-amino-4-hydroxybenzoic acid. The nitro groups on the 3-nitro-4-hydroxybenzoic acids, or salts thereof, may be reduced to an amine by any means known in the art. One particularly useful means is by contacting hydrogen or a source of hydrogen gas with the 3-nitro-4-hydroxybenzoic acid or salt thereof in an aqueous solution in the presence of a hydrogenation catalyst. A preferred class of hydrogenation catalysts are one or more of the Group VIII metal(s) such as palladium and platinum with palladium and platinum being most preferred. It is preferred that the metal(s) be supported. Preferable supports include aluminas, zeolites, silicas, silica gels, silicalite, activated carbons, and diatomaceous earth. More preferred supports are zeolites, silicas, aluminas or activated carbons, with activated carbon being most preferred. The preferred hydrogenation catalyst is palladium-on-carbon. The catalysts may be loaded in any concentration which gives the desired reduction. Preferred catalysts loadings are between about 0.01 and 10 percent by weight, more preferably between about 1.0 and 5.0 with 5.0 percent being most preferred. The reaction pressure is that suitable for reducing the nitro moiety to an amine moiety. Preferred reaction pressures are between about atmospheric and 200 psi (1.4 mPa) with between about atmospheric and 50 psi (100 and 350 kPa) being most preferred. Reaction temperatures are those at which the reaction proceeds, preferred reaction temperatures are between 20°C and 150°C, with between 90°C and 110°C being the most preferred.

Another method of reduction involves contacting the 3-nitro-4-hydroxybenzoic acid salt with aluminum in the zero-valent state, preferably aluminum metal in basic aqueous solution. The amount of aluminum metal is that amount sufficient to result in complete reduction of the nitro moiety to an amine moiety. Preferably aluminum is present in a ratio of aluminum to the 3-nitro-4-hydroxybenzoic acid salt of 2 : 1 or greater; most preferably the aluminum metal is present in a ratio of aluminum metal to 3-nitro-4-hydroxybenzoic acid salt of between 2.2: 1 and 3 : 1. This contacting can be performed at any temperature at which the reduction occurs. Preferred

temperatures are between 15°C and 100°C, with most preferred temperatures being between 25°C and 75°C. This process may take place at any pressure, preferred pressure is atmospheric. The 3-amino-4-hydroxybenzoic acid may be recovered as a salt of the amine by contacting the reaction solution with a strong protic mineral acid, or a source thereof. Generally, a sufficient amount of strong protic mineral acid is added to render the normality of the solution between about 3 and 12, preferably between about 3 and 6. In this embodiment, the desired amine salt precipitates from solution and can be recovered by filtration. The product may thereafter be purified by recrystallization.

In that embodiment where aluminum in a zero-valent state is used to reduce the nitro moiety, the 3-nitro-4-hydroxybenzoic acid, or salt thereof, need not be recovered from the reaction solution of step 2. In fact, such reaction solution can immediately be contacted with the aluminum reduction catalyst so as to result in the reduction of the nitro group to the amine. After such reduction has been completed, then the product can be recovered as described hereinbefore.

Suitable strong protic mineral acids include hydrochloric acid, hydrobromic acid, hydriodic acid, nitric acid, sulfuric acid, and the like. The most preferred acid is hydrochloric acid. The strong protic mineral acid may be added in concentrated form or may be added to the solution or may be added in the nonacidic form, for example, hydrogen chloride may be bubbled through the reaction solution so as to render the reaction solution between 3N and 12N.

$R^1$ is preferably hydrogen, $C_{1-10}$ alkyl, more preferably hydrogen, $C_{1-3}$ alkyl, and most preferably hydrogen. $R_2$ is preferably $C_{1-10}$ alkyl, and most preferably $C_{1-3}$ alkyl. Preferably a is 0 or 1, and most preferably 0. X is preferably chlorine, bromine, or iodine, more preferably chlorine or bromine, and most preferably chlorine. Z is preferably H or an alkali metal cation, and more preferably H, a sodium cation or potassium cation.

One significant advantage of this process is that there is no need to purify the products of the intermediate steps. Furthermore, the product of the final step can easily be purified by one simple recrystallization.

Typical yields are in excess of about 90 mole percent based on starting p-halobenzoic acid or ester thereof. Preferred yields are in excess of about 95 mole percent. Typical purity of 3-amino-4-hydroxybenzoic acid is in excess of 95 weight percent, more preferably 99 weight percent and most preferably 99.95 weight percent.

The following examples are included for illustrative purposes only and are not intended to limit the scope of the invention. Unless otherwise indicated, all parts and percentages are by weight.

**Example 1**

Preparation of 3-amino-4-hydroxybenzoic acid hydrochloride from 4-chlorobenzoic acid.

A - Preparation of 4-chloro-3-nitrobenzoic acid.

To a 2-liter, 3-necked, round-bottom flask is added 680 ml of concentrated $H_2SO_4$ and 400 g of p-chlorobenzoic acid. The mixture is stirred and brought to 0°C by means of a constant temperature bath. A solution of concentrated $HNO_3$ (216 ml) and concentrated $H_2SO_4$ (216 ml) is added dropwise to the reaction mixture at such a rate as to maintain the temperature between 10°C and 25°C. Upon completion of the addition, the reaction temperature is raised to 37°C and the mixture is allowed to stir for a period of 10 - 14 hours. The reaction mixture is then poured over crushed ice and the product, 4-chloro-3-nitrobenzoic acid, is filtered and dried, having a yield of 525.7 g (98.7 percent) with a melting point of 178°C - 180°C. This material is used without further purification.

B - Preparation of 4-hydroxy-3-nitrobenzoic acid.

To a 5-liter, 3-necked, round-bottom flask is added 532 g of NaOH in 3 liters of water and 520 g of 4-chloro-3-nitrobenzoic acid. The solution is heated to reflux (100°C) under nitrogen and stirred for 10 hours. Upon completion, the reaction is cooled to room temperature and neutralized with concentrated hydrochloric acid. The product is isolated by filtration, washed with 3 liters of cold water and dried in a vacuum oven at 30°C overnight at 3 mm Hg. The yield is 430 g (90 percent) of 4-hydroxy-3-nitrobenzoic acid with a melting point of 182°C - 183°C.

C - Preparation of 3-amino-4-hydroxybenzoic acid hydrochloride.

To a 5-liter, 3-necked, round-bottom flask is added 160 g of 4-hydroxy-3-nitrobenzoic acid, 150 ml concentrated hydrochloric acid, 3 liters of distilled water and 25 g of 5 percent palladium on carbon. The reaction mixture is heated to 95°C with vigorous stirring and hydrogen gas is passed into the reaction mixture. Upon completion, the reaction is cooled to room temperature under nitrogen gas and the catalyst is recovered by filtration. The resulting solution is poured into two 4-liter beakers and acidified with concentrated hydrochloric acid to a normality of 4 and allowed to cool to 0°C. The resulting solid is isolated by filtration and dried under vacuum to afford 176 g of crude 3-amino-4-hydroxybenzoic acid hydrochloride monohydrate with a melting point of 300°C decomposition.

D - Recrystallization of 3-amino-4-hydroxybenzoic acid hydrochloride. The product obtained in the previous reaction is placed in a 2-liter, round-bottom flask containing 800 ml of water, 17.6 g of $SnCl_2$ $2H_2O$ 535 ml of concentrated hydrochloric acid and 20 g of decolorizing carbon. The mixture is heated to reflux and kept for a

period of 15 minutes, after which time the carbon is removed by filtration and the solution is cooled slowly to 0°C. The 3-amino-4-hydroxybenzoic acid hydrochloride is isolated by filtration as the monohydrate in 90 mole percent recovered yield (162 g) with a melting point of 300°C decomposition.

**Example 2**

- Preparation of 3-amino-4-hydroxybenzoic acid hydrochloride from 4-chloromethylbenzoate

A - Preparation of 4-chloro-3-nitromethylbenzoate

To a one-liter, 3-necked, round-bottom flask is charged 130 g of p-chloromethylbenzoate in 220 ml of $H_2SO_4$. The solution is cooled to -5°C by means of a constant temperature bath and kept under nitrogen. A solution consisting of 70 ml of concentrated $HNO_3$ and 70 ml of concentrated $H_2SO_4$ is added dropwise at such a rate as to maintain the temperature of the reaction below 15°C. When the addition is completed, the reaction mixture is poured over crushed ice (500 g). The resulting precipitate is isolated by filtration and washed with 300 ml of cold water and dried to afford 160 g of 4-chloro-3-nitromethylbenzoate in 99 mole percent yield having a melting point of 78°C - 80°C. This material is used without further purification.

B - Preparation of 4-hydroxy-3-nitrobenzoic acid.

A 5-liter, 3-necked, round-bottom flask is charged with 432 g of NaOH, 3 liters of $H_2O$ and 320 g of 4-chloro-3-nitromethylbenzoate. The reaction mixture is heated to 95°C for a period of 4 hours under a nitrogen atmosphere. After this time, the reaction is poured over 500 g of crushed ice and 750 ml of concentrated hydrochloric acid. The precipitate which forms is isolated by filtration, washed with 500 ml of cold water and dried at 30°C under vacuum at 3 mm Hg. The resulting 4-hydroxy-3-nitrobenzoic acid is isolated in 91 mole percent yield (245 g) having a melting point of 182°C - 183°C.

C - Preparation of 3-amino-4-hydroxybenzoic acid hydrochloride.

To a 5-liter, 3-necked, round-bottom flask is added 160 g of 4-hydroxy-3-nitrobenzoic acid, 150 ml hydrogen chloride, 3 liter of distilled water and 25 g of 5 percent palladium on carbon. The reaction mixture is heated to 95°C with vigorous stirring and hydrogen gas is passed into the reaction mixture. Upon completion, the reaction is cooled to room temperature under nitrogen gas and the catalyst is recovered by filtration. The resulting solution is poured into two 4-liter beakers and acidified with concentrated hydrochloric acid to a normality of 4 and allowed to cool to 0°C. The resulting solid is isolated by filtration and dried under vacuum to afford 176 g of crude 3-nitro-4-hydroxybenzoic acid hydrochloride monohydrate with a melting point of 300°C decomposition.

D - Recrystallization of 3-amino-4-hydroxybenzoic acid hydrochloride.

The product obtained in the previous reaction is placed in a 2-liter, round-bottom flask containing 800 ml of water, 17.6 g of $SnCl_2$ $2H_2O$, 535 ml of concentrated hydrochloric acid and 20 g of decolorizing carbon. The mixture is heated to reflux and kept for a period of 15 minutes, after which time the carbon is removed by filtration and the solution is cooled slowly to 0°C. The 3-amino-4-hydroxybenzoic acid hydrochloride is isolated by filtration as the monohydrate in 95 mole percent recovered yield (162 g) with a melting point of 300°C decomposition.

**Example 3**

A - Preparation of 3-amino-4-hydroxybenzoic acid hydrochloride by aluminum reduction.

4-Chloro-3-nitrobenzoic acid is prepared as described in Examples 1A and 1B above.

B - Preparation of 3-amino-4-hydroxybenzoic acid hydrochloride

A 5-liter, 3-necked flask is charged with 227 g of 4-chloro-3-nitrobenzoic acid, 3 liters of water and 250 g of solid KOH, and heated to reflux under a nitrogen atmosphere. The reaction is maintained at this temperature for a period of $9^1/_2$ hours after which time the reaction is cooled to 70°C. Aluminum metal (67 g) is slowly added to the reaction at such a rate as to maintain the temperature at 95°C. Upon completion of this addition, the reaction mixture is filtered and the aqueous portion is acidified with concentrated hydrogen chloride and allowed to cool to 0°C. The crude 3-amino-4-hydroxybenzoic acid hydrochloride is isolated by filtration and dried. The yield of product is 222 g or 91 mole percent as the monohydrate.

C - Recrystallization of 3-amino-4-hydroxybenzoic acid hydrochloride.

Recrystallization of 3-amino-4-hydroxybenzoic acid hydrochloride is accomplished as described in Examples 1 and 2 with a recovery of 202 g or 91 mole percent, based on the amount charged, having a melting point of 300°C with decomposition.

**Example 4**

To a 5-liter, 3-necked, round-bottom flask, equipped with a stirrer and thermometer, is added 2.0 liters of concentrated $H_2SO_4$ and 800 g of powdered (1 - 2 micrometers) p-chlorobenzoic acid. The mixture is stirred and cooled to 0°C in an oil bath. To the cooled mixture is added 432 ml of 71 weight percent nitric acid in water solution dropwise. The nitric acid solution is added at a rate slow enough to maintain the temperature lower than about 30°C. Upon completion of adding nitric acid, the temperature is raised to 37°C over 3 hours. The reaction mixture is then cooled to 15°C. To the cooled mixture is added 0.75 liter of room temperature water at a rate so as to keep the reaction temperature below about 40°C. The product is isolated by filtration using fritted glass funnel and washed with 1.5 liters of room temperature water. The washed product is air-dried until a constant weight is observed to yield 1.0174 kg of 4-chloro-3-nitrobenzoic acid, which has a melting point of 178°C - 179.5°C. This is a yield of 99 mole percent. This material can be used without further purification.

**Claims**

1. A process for the preparation of a 3-amino-4-hydroxybenzoic acid which comprises

(a) contacting a p-halobenzoic acid or ester thereof, which is unsubstituted or substituted with one or two alkyl groups, with the proviso that at least one of the positions ortho to the halo moiety is unsubstituted, with nitric acid in an acidic reaction medium under conditions such that a 3-nitro-4-halobenzoic acid or ester thereof is prepared;

(b) contacting the 3-nitro-4-halobenzoic acid or ester thereof with at least a two equivalent excess of an alkali metal hydroxide in a reaction medium under conditions such that the halo moiety is replaced with a hydroxide moiety or a salt thereof, to prepare a 3-nitro-4-hydroxybenzoic acid, or salt thereof; and

(c) reducing the 3-nitro-4-hydroxybenzoic acid or salt thereof in a reaction medium under conditions such that a 4-hydroxy-3-aminobenzoic acid is recovered in purity in excess of 95 weight percent.

2. A process for the preparation of a 3-amino-4-hydroxybenzoic acid by reducing a 3-nitro-4-hydroxy-benzoic acid or salt thereof in a reaction medium under conditions such that a 3-amino-4-hydroxybenzoic acid is prepared, characterised in that said 3-nitro-4-hydroxybenzoic acid or salt thereof has been obtained by:

(a) contacting a p-halobenzoic acid or ester thereof, which is unsubstituted or substituted with one or two alkyl groups, with the proviso that at least one of the positions ortho to the halo moiety is unsubstituted, with nitric acid in an acidic reaction medium under conditions such that a 3-nitro-4-halobenzoic acid or ester thereof is prepared; and

(b) contacting the 3-nitro-4-halobenzoic acid or ester thereof with at least a two equivalent excess of an alkali metal hydroxide in a reaction medium under conditions such that the halo moiety is replaced with a hydroxide moiety or a salt thereof, to prepare the 3-nitro-4-hydroxybenzoic acid, or salt thereof and in that the 3-amino-4-hydroxybenzoic acid is recovered in purity in excess of 95 weight percent.

3. A process as claimed in any one of the preceding claims wherein the p-halobenzoic acid or ester thereof corresponds to the formula

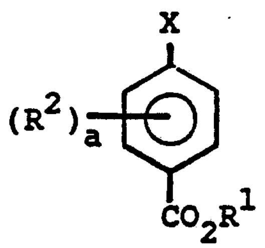
,

(I)

the 3-nitro-4-halobenzoic acid or ester thereof corresponds to the formula

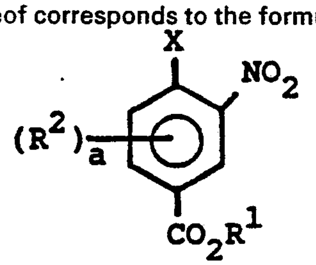
,

(II)

the 3-nitro-4-hydroxybenzoic acid or salt thereof corresponds to the formula

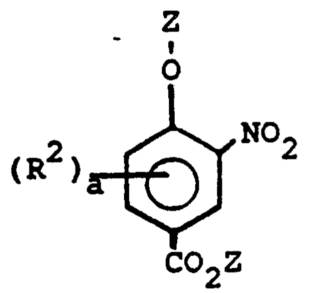

(III)

and the 3-amino-4-hydroxybenzoic acid corresponds to the formula

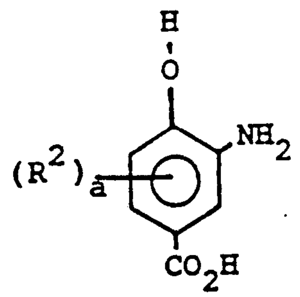

(IV)

wherein
$R^1$ is hydrogen or $C_1$-$C_{10}$ alkyl;
$R^2$ is independently in each occurrence $C_1$-$C_{10}$ alkyl;
X is a halogen
Z is independently in each occurrence hydrogen or a cation; and
a is 0,1 or 2.

4. A process as claimed in claim 3, wherein
$R^1$ is hydrogen or $C_1$-$C_3$ alkyl;
$R^2$ is $C_1$-$C_3$ alkyl;
X is chlorine, bromine or iodine;
Z is hydrogen or an alkali metal cation; and
a is 0 or 1.

5. A process as claimed in claim 4, wherein $R^1$ is hydrogen; X is chlorine; Z is sodium; and a is 0.

6. A process as claimed in any one of the preceding claims, wherein the said acidic medium contains more than 20 weight percent of a Lewis or Bronsted acid.

7. A process as claimed in claim 6, wherein the said amount of said acid is more than 50 weight percent of the aqueous medium.

8. A process as claimed in claim 7, wherein the said amount of said acid is more than 70 weight percent of the aqueous medium.

9. A process as claimed in claim 8, wherein the said amount of said acid is more than 90 percent of the aqueous medium."

10. A process as claimed in any one of the preceding claims, wherein each equivalent of p-halobenzoic acid or ester thereof is contacted with at least 1 equivalent of nitric acid.

11. A process as claimed in claim 10, wherein each equivalent of p-halobenzoic acid or ester thereof is contacted with in excess of 1 equivalent of sulfuric acid.

12. A process as claimed in claim 11, wherein the ratio of sulfuric acid to p-halobenzoic acid or ester thereof is at least 1.3 : 1.

13. A process as claimed in any one of the preceding claims, wherein the said excess of alkali metal hydroxide is 3.5 to 4.5 equivalents when reacted with a 3-nitro-4-halobenzoic acid or 4.5 to 5.5 equivalents when reacted with a 3-nitro-4-halobenzoic acid ester.

14. A process as claimed in any one of the preceding claims, wherein the temperature in step (a) is between -10°C and 50°C.

15. A process as claimed in any one of the preceding claims, wherein the temperature in step (b) is between 65°C and 120°C.

16. A process as claimed in claim 15, wherein said temperature is between 90°C and 110°C.

17. A process as claimed in claim 16, wherein step (b) is carried out at reflux in water.

18. A process as claimed in any one of the preceding claims, wherein the 3-nitro-4-hydroxybenzoic acid is reduced by contacting with hydrogen gas or a source of hydrogen in the presence of a heterogeneous reduction catalyst.

19. A process as claimed in claim 18, wherein the heterogeneous reduction catalyst is palladium-on-charcoal.

20. A process as claimed in any one of claims 1 to 16, wherein the 3-nitro-4-hydroxybenzoic acid is reduced by contacting with aluminum in the zero-valent state.

21. A process as claimed in claim 20, wherein the aluminum in the zero-valent state is contacted with the 3-nitro-4-hydroxybenzoic acid in the reaction medium of step (b).

22. A process as claimed in any one of the preceding claims, wherein the alkali metal salt of 3-nitro-4-hydroxybenzoic acid is recovered from the reaction medium of step (b) by contacting the reaction medium with a strong protic acid so that the 3 nitro-4-hydroxybenzoic acid precipitates from the reaction medium.

23. A process as claimed in claim 22, wherein the strong protic acid is hydrochloric acid.

24. A process as claimed in any one of the preceding claims, wherein the 3-amino-4-hydroxybenzoic acid is recovered as an amine salt, after reduction by contacting the reaction medium with a sufficient amount of a strong protic mineral acid, to convert the 3-amino-4-hydroxybenzoic acid to an amine salt, and precipitate said salt.

25. A process for the preparation of an AB polybenzoxazole from a 3-amino-4-hydroxybenzoic acid characterized in that said 3-amino-4-hydroxybenzoic acid is prepared by a process as claimed in any one of the preceding claims.

**Patentansprüche**

1. Verfahren zum Herstellen einer 3-Amino-4-hydroxybenzoesäure durch

(a) Inberührungbringen einer p-Halogenbenzoesäure oder Ester derselben, die nicht substituiert ist oder mit ein oder zwei Alkylgruppen substituiert ist, unter der Bedingung, daß mindestens eine der Orthostellungen zur Halogengruppe nicht substituiert ist, mit Salpetersäure in saurem Reaktionsmedium unter solchen Bedingungen, daß eine 3-Nitro-4-halogenbenzoesäure oder deren Ester gebildet wird,

(b) Inberührungbringen der 3-Nitro-4-halogenbenzoesäure oder deren Ester mit mindestens zwei Äquivalenten Überschuß eines Alkalihydroxids in einem Reaktionsmedium unter solchen Bedingungen, daß das Halogen durch eine Hydroxygruppe oder ein Salz derselben ersetzt wird, um eine 3-Nitro-4-hydroxybenzoesäure oder ein Salz derselben auszubilden, und

(c) Reduzieren der 3-Nitro-4-hydroxybenzoesäure oder deren Salz in einem Reaktionsmedium unter solchen Bedingungen, daß eine 4-Hydroxy-3-aminobenzoesäure in einer Reinheit von mehr als 95 Gew.- % gewonnen wird.

2. Verfahren zum herstellen einer 3-Amino-4-hydroxybenzoesäure durch Reduzieren einer 3-Nitro-4-hydroxybenzoesäure oder eines Salzes derselben in einem Reaktionsmedium unter solchen Bedingungen, daß eine 3-Amino-4-hydroxybenzoesäure gebildet wird,

dadurch gekennzeichnet, daß die 3-Nitro-4-hydroxybenzoesäure oder das Salz derselben erhalten wurde durch:

(a) Inberührungbringen einer p-Halogenbenzoesäure oder Ester derselben, die nicht substituiert ist oder substituiert ist mit einer oder zwei Alkylgruppen, mit der Bedingung, daß mindestens eine der Orthostellungen zur Halogengruppe nicht substituiert ist, mit Salpetersäure in einem sauren Reaktionsmedium unter solchen Bedingungen, daß eine 3-Nitro-4-halogenbenzoesäure oder deren Ester gebildet wird, und

(b) Inberührungbringen der 3-Nitro-4-halogenbenzoesäure oder deren Ester mit mindestens zwei Äquivalenten Überschuß eines Alkalihydroxids in einem Reaktionsmedium under solchen Bedingungen, daß Halogen durch eine Hydroxygruppe oder ein Salz derselben ersetzt wird, um eine 3-Nitro-4-hydroxybenzoesäure oder deren Salz zu bilden

und daß die 3-Amino-4-hydroxybenzoesäure in einer Reinheit von mehr als 95 Gew.- % gewonnen wird.

3. Verfahren nach jedem der vorstehenden Ansprüche,

dadurch gekennzeichnet, daß die p-Halogenbenzoesäure oder deren Ester der Formel entspricht

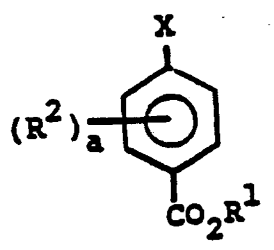

(I)

die 3-Nitro-4-halogenbenzoesäure oder deren Ester der Formel entspricht

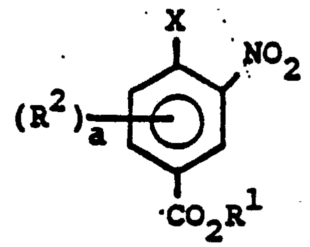

(II)

die 3-Nitro-4-hydroxybenzoesäure oder deren Salz der Formel entspricht

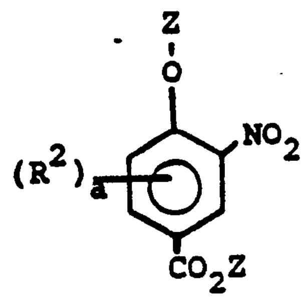

(III)

und die 3-Amino-4-hydroxybenzoesäure der Formel entspricht

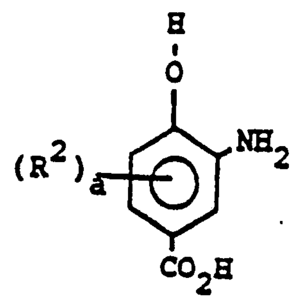

(IV)

in denen
$R^1$ Wasserstoff oder $C_1C_{10}$ Alkyl ist,
$R^2$ unabhängig bei jedem Auftreten $C_1$-$C_{10}$ Alkyl ist,
X halogen ist,
Z unabhängig bei jedem Auftreten Wasserstoff oder ein Kation ist, und
a 0, 1 oder 2 ist.

4. Verfahren nach Anspruch 3,
dadurch gekennzeichnet, daß
$R^1$ Wasserstoff oder $C_1$-$C_3$ Alkyl ist,
$R^2$ $C_1$-$C_3$ Alkyl ist,
X Chlor, Brom oder Jod ist,
Z Wasserstoff oder ein Alkalikation ist, und
a 0 oder 1 ist.

5. Verfahren nach Anspruch 4,
dadurch gekennzeichnet, daß $R^1$ Wasserstoff, X Chlor, Z Natrium und a 0 ist.

6. Verfahren nach jedem der vorstehenden Ansprüche,
dadurch gekennzeichnet, daß das saure Medium mehr als 20 Gew.- % einer Lewis- oder Bronsted-Säure enthält.

7. Verfahren nach Anspruch 6,
dadurch gekennzeichnet, daß die Säuremenge mehr als 50 Gew.- % des wässrigen Mediums beträgt.

8. Verfahren nach Anspruch 7,
dadurch gekennzeichnet, daß die Säuremenge mehr als 70 Gew.- % des wässrigen Mediums beträgt.

9. Verfahren nach Anspruch 8,
dadurch gekennzeichnet, daß die Säuremenge mehr als 90 % des wässrigen Mediums beträgt.

10. Verfahren nach jedem der vorstehenden Ansprüche,
dadurch gekennzeichnet, daß jedes Äquivalent von p-Halogenbenzoesäure oder deren Ester mit mindesten einem Äquivalent Salpetersäure in Berührung gebracht wird.

11. Verfahren nach Anspruch 10,
dadurch gekennzeichnet, daß jedes Äquivalent von p-Halogenbenzoesäure oder des Esters derselben mit

einem Überschuß von einem Äquivalent Schwefelsäure in Berührung gebracht wird.

12. Verfahren nach Anspruch 11,
dadurch gekennzeichnet, daß das Verhältnis von Schwefelsäure zu p-Halogenbenzoesäure oder deren Ester mindestens 1, 3 : 1 beträgt.

13. Verfahren nach jedem der vorstehenden Ansprüche,
dadurch gekennzeichnet, daß der Überschuß an Alkalihydroxid 3,5 - 4,5 Äquivalente beträgt, wenn mit einer 3-Nitro-4-halogenbenzoesäure umgesetzt wird, oder 4,5 - 5,5 Äquivalente beträgt, wenn mit einem 3-Nitro-4-halogenbenzoesäureester umgesetzt wird.

14. Verfahren nach jedem der vorstehenden Ansprüche,
dadurch gekennzeichnet, daß die Temperatur in Schritt (a) zwischen -10° C und 50° C beträgt.

15. Verfahren nach jedem der vorstehenden Ansprüche,
dadurch gekennzeichnet, daß die Temperatur in Schritt (b) zwischen 65° C und 120° C beträgt.

16. Verfahren nach Anspruch 15,
dadurch gekennzeichnet, daß die Temperatur zwischen 90° C und 110° C beträgt.

17. Verfahren nach Anspruch 16,
dadurch gekennzeichnet, daß der Schritt (b) unter Rückfluß in Wasser ausgeführt wird.

18. Verfahren nach jedem der vorstehenden Ansprüche,
dadurch gekennzeichnet, daß die 3-Nitro-4-hydroxybenzoesäure reduziert wird durch Inberührungbringen mit Wasserstoffgas oder einer Wasserstoffquelle in Gegenwart eines heterogenen Reduktionskatalysators.

19. Verfahren nach Anspruch 18,
dadurch gekennzeichnet, daß der heterogene Reduktionskatalysator Palladium auf Holzkohle ist.

20. Verfahren nach jedem der Ansprüche 1 - 16,
dadurch gekennzeichnet, daß die 3-Nitro-4-hydroxybenzoesäure reduziert wird durch Inberührungbringen mit Aluminium im nullwertigen Zustand.

21. Verfahren nach Anspruch 20,
dadurch gekennzeichnet, daß Aluminium im nullwertigen Zustand in Berührung gebracht wird mit der 3-Nitro-4-hydroxybenzoesäure in dem Reaktionsmedium von Schritt (b).

22. Verfahren nach jedem der vorstehenden Ansprüche,
dadurch gekennzeichnet, daß das Alkalisalz von 3-Nitro-4-hydroxybenzoesäure gewonnen wird aus der Reaktionsmedium von Schritt (b) durch Inberührungbringen des Reaktionsmediums mit einer stark protischen Säure, so daß die 3-Nitro-4-hydroxybenzoesäure aus dem Reaktionsmedium ausfällt.

23. Verfahren nach Anspruch 22,
dadurch gekennzeichnet, daß die stark protische Säure Chlorwasserstoffsäure ist.

24. Verfahren nach jedem der vorstehenden Ansprüche,
dadurch gekennzeichnet, daß die 3-Amino-4-hydroxybenzoesäure gewonnen wird als Aminsalz nach Reduktion durch Inberührungbringen des Reaktionsmediums mit einer ausreichenden Menge einer stark protischen Mineralsäure, um die 3-Amino-4-hydroxybenzoe-säure in ein Aminsalz umzuwandeln, und Ausfällen des Salzes.

25. Verfahren zur Herstellung eines AB Polybenzooxazols aus 3-Amino-4-hydroxybenzoesäure,
dadurch gekennzeichnet, daß die 3-Amino-4-hydroxybenzoesäure hergestellt wird durch ein Verfahren nach jedem der vorstehenden Ansprüche.

## Revendications

1. Procédé de préparation d'un acide amino-3 hydroxy-4 benzoïque qui consiste:

(a) à mettre un acide p-halobenzoïque ou un ester de celui-ci, acide ou ester qui n'est pas substitué ou est substitué par un ou deux radicaux alcoyle à condition qu'au moins l'une des positions ortho par rapport l'halogène à ne soit pas substituée, en contact avec de l'acide nitrique dans un milieu acide de réaction se trouvant dans des conditions telles qu'un acide nitro-3 halo-4 benzoïque ou un ester de celui-ci soit préparé,

(b) à mettre cet acide nitro-3 halo-4 benzoïque ou l'ester de celui-ci en contact avec un excès d'au moins deux équivalents d'un hydroxyde de métal alcalin dans un milieu de réaction se trouvant dans des conditions telles que l'halogène soit remplacé par un radical hydroxyle ou un sel de celui-ci, de façon à préparer un acide nitro-3 hydroxy-4 benzoïque ou un sel de celui-ci et

(c) à réduire cet acide nitro-3 hydroxy-4 benzoïque ou le sel de celui-ci dans un milieu de réaction se trouvant dans des conditions telles qu'on récupère un acide amino-3 hydroxy-4 benzoïque avec une pureté dépassant 95 % en poids.

2. Procédé de préparation d'un acide amino-3 hydroxy-4 benzoïque par réduction d'un acide nitro-3 hydroxy-4 benzoïque ou d'un sel de celui-ci dans un milieu de réaction se trouvant dans des conditions telles qu'un acide amino-3 hydroxy-4 benzoïque soit préparé, caractérisé en ce que l'acide nitro-3 hydroxy-4 benzoïque ou le sel de celui-ci a été obtenu:

(a) en mettant un acide p-halobenzoïque ou un ester de celui-ci, acide ou ester qui n'est pas substitué ou est substitué par un ou deux radicaux alcoyle à condition qu'au moins l'une des positions ortho par rapport l'halogène à ne soit pas substituée, en contact avec de l'acide nitrique dans un milieu acide de réaction se

trouvant dans des conditions telles qu'un acide nitro-3 halo-4 benzoïque ou un ester de celui-ci se trouve préparé et

(b) en mettant cet acide nitro-3 halo-4 benzoïque ou l'ester de celui-ci en contact avec un excès d'au moins deux équivalents d'un hydroxyde de métal alcalin dans un milieu de réaction se trouvant dans des conditions telles que l'halogène se trouve remplacé par un radical hydroxyle ou un sel de celui-ci, de façon à préparer un acide nitro-3 hydroxy-4 benzoïque ou un sel de celui-ci et en ce qu'on récupère l'acide amino-3 hydroxy-4 benzoïque sous une pureté dépassant 95 % en poids.

3. Procédé suivant l'une quelconque des revendications précédentes, selon lequel l'acide p-halobenzoïque ou l'ester de celui-ci correspond à la formule

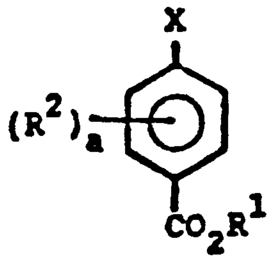

(I)

l'acide nitro-3 halo-4 benzoïque ou son ester correspond à la formule

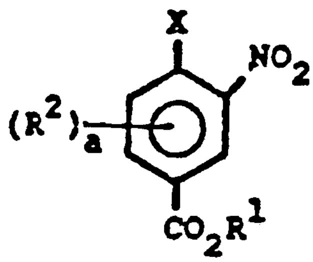

(II)

l'acide nitro-3 hydroxy-4 ou son sel correspond à la formule

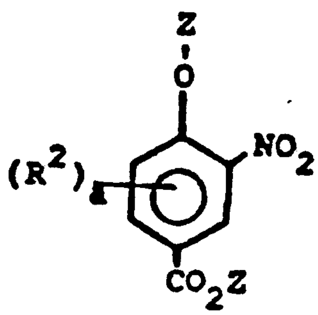

(III)

et l'acide amino-3 hydroxy-4 benzoïque correspond à la formule

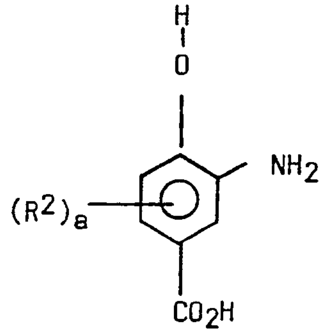

(IV)

dans lesquelles

$R^1$ est l'hydrogène ou un alcoyle en $C_1$-$C_{10}$,
$R^2$ est, d'une manière indépendante et dans chaque cas, un alcoyle en $C_1$-$C_{10}$,
X est un halogène,
Z est, d'une manière indépendante dans chaque cas, l'hydrogène ou un cation et
a vaut 0, 1 ou 2.

4. Procédé suivant la revendication 3, selon lequel:
$R^1$ est l'hydrogène ou un alcoyle en $C_1$-$C_3$,
$R^2$ est un alcoyle en $C_1$-$C_3$,
X est le chlore, le brome ou l'iode,
Z est l'hydrogène ou un cation de métal alcalin et
a vaut 0 ou 1.

5. Procédé suivant la revendication 4, selon lequel $R^1$ est l'hydrogène, X est le chlore, Z est le sodium et a vaut 0.

6. Procédé suivant l'une quelconque des revendications précédentes, selon lequel le milieu acide contient plus de 20 % en poids d'un acide de Lewis ou de Bronsted.

7. Procédé suivant la revendication 6, selon lequel la quantité de l'acide représente plus de 50 % en poids du milieu aqueux.

8. Procédé suivant la revendication 7, selon lequel la quantité de l'acide représente plus de 70 % en poids du milieu aqueux.

9. Procédé suivant la revendication 8, selon lequel la quantité de l'acide représente plus de 90 % du milieu aqueux.

10. Procédé suivant l'une quelconque des revendications précédentes, selon lequel on met chaque équivalent d'acide p-halobenzoïque ou d'ester de celui-ci en contact avec au moins 1 équivalent d'acide nitrique.

11. Procédé suivant la revendication 10, selon lequel on met en contact chaque équivalent d'acide p-halobenzoïque ou d'ester de celui-ci avec un excès de l'équivalent d'acide sulfurique.

12. Procédé suivant la revendication 11, selon lequel le rapport de l'acide sulfurique à l'acide p-halobenzoïque ou ester de celui-ci vaut au moins 1,3 : 1.

13. Procédé suivant l'une quelconque des revendications précédentes, selon lequel l'excès de l'hydroxyde de métal alcalin est de 3,5 à 4,5 équivalents lorsqu'on le fait réagir avec un acide nitro-3 halo-4 benzoïque, ou de 4,5 à 5,5 équivalents lorsqu'on le fait réagir avec un ester d'acide nitro-3 halo-4 benzoïque.

14. Procédé suivant l'une quelconque des revendications précédentes, selon lequel la température de l'étape (a) est comprise entre -10° C et 50° C.

15. Procédé suivant l'une quelconque des revendications précédentes, selon lequel la température de l'étape (b) est comprise entre 65° C et 120° C.

16. Procédé suivant la revendication 15, selon lequel la température est comprise entre 90° C et 110° C.

17. Procédé suivant la revendication 16, selon lequel on conduit l'étape (b) au reflux dans l'eau.

18. Procédé suivant l'une quelconque des revendications précédentes, selon lequel on réduit l'acide nitro-3 hydroxy-4 benzoïque en le mettant en contact avec de l'hydrogène gazeux ou une source d'hydrogène, en présence d'un catalyseur de réduction de type hétérogène.

19. Procédé suivant la revendication 18, selon lequel le catalyseur de réduction de type hétérogène est du palladium sur charbon.

20. Procédé suivant l'une quelconque des revendications 1 à 16, selon lequel on réduit l'acide nitro-3 hydroxy-4 benzoïque en le mettant en contact avec l'aluminium à l'état de valence zéro.

21. Procédé suivant la revendication 20, selon lequel on met l'aluminium à l'état de valence zéro en contact avec l'acide nitro-3 hydroxy-4 benzoïque dans le milieu de réaction de l'étape (b).

22. Procédé suivant l'une quelconque des revendications précédentes, selon lequel on récupère le sel de métal alcalin de l'acide nitro-3 hydroxy-4 benzoïque à partir du milieu de réaction de l'étape (b) en mettant ce milieu de réaction en contact avec un acide protique fort de façon que l'acide nitro-3 hydroxy-4 benzoïque précipite à partir de ce milieu de réaction.

23. Procédé suivant la revendication 22, selon lequel l'acide protique fort est l'acide chlorhydrique.

24. Procédé suivant l'une quelconque des revendications précédentes, selon lequel on récupère l'acide amino-3 hydroxy-4 benzoïque sous la forme d'un sel d'amine, après réduction par mise en contact du milieu de réaction avec une quantité d'un acide minéral protique fort qui est suffisante pour convertir l'acide amino-3 hydroxy-4 benzoïque en un sel d'amine et pour précipiter ce sel.

25. Procédé de préparation d'un polybenzoxazole AB à partir d'un acide amino-3 hydroxy-4 benzoïque, caractérisé en ce que l'acide amino-3 hydroxy-4 benzoïque est préparé par un procédé suivant l'une quelconque des revendications précédentes.